# EUROPEAN PATENT APPLICATION

(11) **EP 4 245 835 A1**
(43) Date of publication of application: **20.09.2023**
(21) Application number: 21891440.6
(22) Date of filing: 18.08.2021
(51) Int. Cl.: C12M 1/00, C12P 5/02, C12N 1/20, C12Q 1/6851, C02F 11/04

(54) **LIGNOCELLULOSE DECOMPOSITION SYSTEM AND LIGNOCELLULOSE DECOMPOSITION METHOD**

(30) Priority: 13.11.2020 JP 2020189313
(71) Applicant: Tohoku University, Sendai-shi, Miyagi 980-8577 (JP)
(72) Inventor: AKASHI, Akira, Kobe-shi, Hyogo 651-0072 (JP); TADA, Chika, Sendai-shi, Miyagi 980-8577 (JP); ENDO, Masaya, Sendai-shi, Miyagi 980-8577 (JP)
(74) Representative: TBK
(86) International application number: PCT/JP2021/030266
(87) International publication number: WO 2022/102192

(57) **Abstract**

Provided are a lignocellulose degradation system and a method for degrading lignocellulose that can efficiently degrade lignocellulosic biomass. The method includes: at least one of (a) selecting a rumen fluid derived from a ruminant using abundance of a specific bacterium as an indicator, (b) controlling culture of rumen microorganisms using abundance of a specific bacterium as an indicator, or (c) controlling lignocellulose degradation using abundance of a specific bacterium as an indicator; and (d) performing methane fermentation using as a substrate a lignocellulose degradation product produced by degrading lignocellulosic biomass using at least one of the rumen fluid selected in step (a), a culture fluid obtained in step (b), or a reaction liquid obtained in step (c).

## Description

### Technical Field

The present invention relates to a lignocellulose degradation system and a method for degrading lignocellulose that can efficiently degrade lignocellulosic biomass.

### Background Art

Lignocellulosic biomass is composed of cell walls of plant cells, that is, the main component of plant fibers, and is an organic carbon source abundantly present on Earth, thus attracting attention as an energy resource replacing fossil fuels, such as petroleum. Lignocellulosic biomass is difficult to degrade and thus requires significant energy, cost, and time in the degradation stage. The actual situation is that this delays the expansion of its use as an energy resource.

A technique has been known in the art for degrading lignocellulosic biomass by utilizing bacteria contained in rumen fluid present in the paunch (rumen) of ruminants (e.g., Patent Literature 1).

Patent Literature 1 describes an organic acid fermentation method by rumen fluid using lignocellulose-containing waste. Specifically, Patent Literature 1 disclosed that in a pretreatment of methane fermentation using lignocellulosic biomass, such as waste paper, as a raw material, under anaerobic conditions generated by co-existence of cysteine, which has a reducing action, in a reaction system using rumen fluid, a reaction is performed at a medium temperature of 30 to 45°C for a relatively short time of 6 to 24 hours to increase production amounts of organic acids, such as acetic acid, propionic acid, and butyric acid, which can be used as substrates (raw materials) for methane fermentation, and concomitantly, methane is efficiently produced.

### Citation List

### Patent Literature

Patent Literature 1: WO 2012/053631

### Summary of Invention

### Technical Problem

However, Patent Literature 1 does not take into account that the number of bacteria living in rumen fluid is not constant for each individual ruminant from which rumen fluid is collected and that lignocellulolytic activity varies, and does not describe selection of a rumen fluid for stably and efficiently progressing lignocellulose degradation. Some rumen fluids do not have sufficient lignocellulolytic activity, and thus lignocellulose degradation may not proceed well even if the supply amount of rumen fluid is adjusted according to the amount of biomass. In addition, when the production amounts of lignocellulose degradation products decrease, methane fermentation using them as substrates (raw materials) does not proceed either, and thus methane cannot be stably and efficiently produced.

Accordingly, an object of the present invention is to provide a lignocellulose degradation system and a method for degrading lignocellulose for enabling efficient degradation of lignocellulosic biomass and allowing efficient progress of methane fermentation using, as substrates (raw materials), degradation products obtained by degradation of lignocellulosic biomass.

### Solution to Problem

To solve the above problems, a lignocellulose degradation system according to an embodiment of the present invention is a lignocellulose degradation system for degrading lignocellulosic biomass and is characterized by including:
at least one of a selection means for selecting a rumen fluid derived from a ruminant using abundance of a specific bacterium as an indicator, a culture vessel for controlling culture of rumen microorganisms using abundance of a specific bacterium as an indicator, or a degradation vessel for controlling degradation of lignocellulose using abundance of a specific bacterium as an indicator; and
a methane fermenter for performing methane fermentation using as a substrate a lignocellulose degradation product produced by degrading lignocellulosic biomass using at least one of the rumen fluid selected in the selection means, a culture fluid in the culture vessel, or a reaction liquid in the degradation vessel.

According to the above configuration, the system can select a rumen fluid with high lignocellulolytic activity using the number of a specific bacterium as an indicator or evaluate lignocellulolytic activity while monitoring culture of rumen microorganisms using the number of a specific bacterium as an indicator. Thus, degradation of lignocellulosic biomass proceeds efficiently, and this can increase the production amount of a lignocellulose degradation product, such as a monosaccharide, an oligosaccharide, or a volatile fatty acid. Concomitantly, the system can allow methane fermentation using a lignocellulose degradation product as a substrate (raw material) to proceed efficiently in the methane fermenter.

In addition, an embodiment of the present invention is characterized in that in the lignocellulose degradation system, the specific bacterium is at least one or more types of three types of bacteria, *Fibrobacter succinogenes, Ruminococcus albus,* and *Prevotella ruminicola.*

The above configuration enables the system to more accurately evaluate lignocellulolytic activity by specifying three types of bacteria, *Fibrobacter succinogenes, Ruminococcus albus,* and *Prevotella ruminicola,* as bacteria with particularly high lignocellulolytic activity, and using a rumen fluid selected using as an indicator abundance of at least one or more types of these three types of bacteria; or by monitoring culture of rumen microorganisms using abundance of at least one or more types of the three types of bacteria as an indicator. As a result, the system enables degradation of lignocellulosic biomass to proceed stably and efficiently.

In addition, an embodiment of the present invention is characterized in that in the lignocellulose degradation system, the abundance of the specific bacterium as measured by a quantitative PCR method is 1.0 × 10⁹ copies/mL or more for *Fibrobacter succinogenes,* 1.0 × 10⁷ copies/mL or more for *Ruminococcus albus,* and 1.0 × 10¹⁰ copies/mL or more for *Prevotella ruminicola.*

According to the above configuration, a rumen fluid with high lignocellulolytic activity can be obtained by selecting a rumen fluid in which abundance of at least one or more types of the three types of bacteria is not less than a predetermined value. In addition, a culture fluid with high lignocellulolytic activity can be obtained by culturing rumen microorganisms to increase the abundance of at least one or more types of the three types of bacteria to a predetermined value or more in the culture vessel. Furthermore, the system can promote generation amount of methane gas in a downstream methane fermenter by periodically monitoring the three types of bacteria in the culture vessel or the degradation vessel and operating the culture vessel or the degradation vessel to increase the abundance of at least one or more types of those bacteria to a predetermined value or more.

In addition, an embodiment of the present invention is characterized in that in the cellulose degradation system, at least one of the culture of rumen microorganisms in the culture vessel or the degradation of lignocellulosic biomass in the degradation vessel is controlled based on a parameter including temperature, pH, oxidation-reduction potential (ORP), hydraulic retention time (HRT) or solids retention time (SRT), and ammonium nitrogen concentration in a culture medium.

According to the above configuration, the inside of the culture vessel or the inside of the degradation vessel can be approximated to the environment in the paunch (rumen) of ruminants and kept in a condition suitable for degrading lignocellulosic biomass by controlling at least one of the culture of rumen microorganisms in the culture vessel or the degradation of lignocellulosic biomass in the degradation vessel based on a parameter including temperature, pH, oxidation-reduction potential (ORP), hydraulic retention time (HRT) or solids retention time (SRT), and ammonium nitrogen concentration in a culture medium. As a result, the system can degrade lignocellulosic biomass more stably and efficiently.

In addition, an embodiment of the present invention is characterized in that in the cellulose degradation system, the culture vessel or the degradation vessel is a culture and degradation vessel for performing the culture of rumen microorganisms and the degradation of lignocellulose in one vessel.

The above configuration enables the system to perform the culture of rumen microorganisms and the degradation of lignocellulose in one vessel and thus this can reduce the production cost of the lignocellulose degradation system and can make the equipment compact.

To solve the above problems, a method for degrading lignocellulose according to an embodiment of the present invention is a lignocellulose degradation method for degrading lignocellulosic biomass and is characterized by including:
at least one of (a) selecting a rumen fluid derived from a ruminant using abundance of a specific bacterium as an indicator, (b) controlling culture of rumen microorganisms using abundance of a specific bacterium as an indicator, or (c) controlling degradation of lignocellulose using abundance of a specific bacterium as an indicator; and
(d) performing methane fermentation using as a substrate a lignocellulose degradation product produced by degrading lignocellulosic biomass using at least one of the rumen fluid selected in step (a), a culture fluid obtained in step (b), or a reaction liquid obtained in step (c).

According to the above configuration, the method can select a rumen fluid with high lignocellulolytic activity using the number of a specific bacterium as an indicator or evaluate lignocellulolytic activity while monitoring culture of rumen microorganisms using the number of a specific bacterium as an indicator. Thus, degradation of lignocellulosic biomass proceeds efficiently, and this can increase the production amount of a lignocellulose degradation product, such as a monosaccharide, an oligosaccharide, or a volatile fatty acid. Concomitantly, the method can allow methane fermentation using a lignocellulose degradation product as a substrate (raw material) to proceed efficiently in the methane fermenter. As a result, the method can be expected to improve the yield of methane.

### Brief Description of Drawings

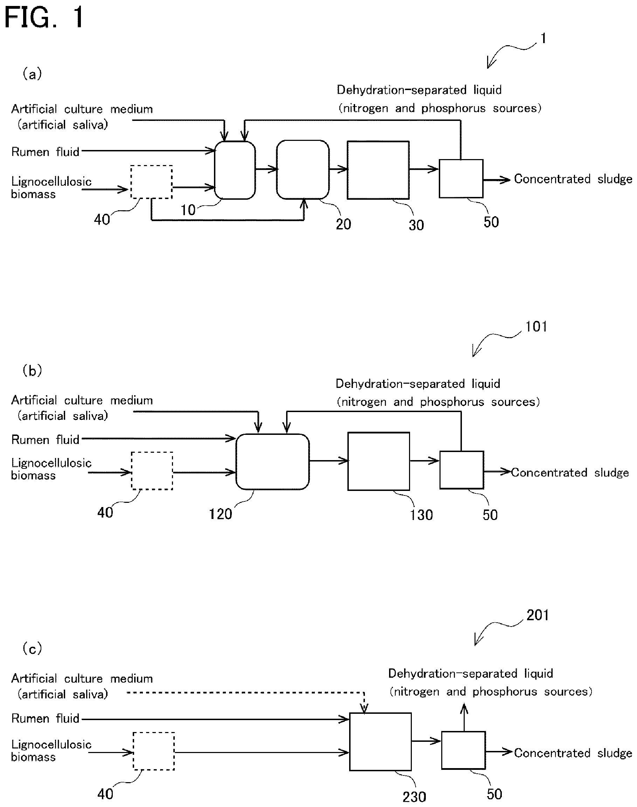

FIG. 1 is a schematic diagram illustrating a lignocellulose degradation system for implementing a lignocellulose degradation method according to the present embodiment. FIG. 1(a) is a diagram illustrating a lignocellulose degradation system constituted of a three-phase process, FIG. 1(b) is a diagram illustrating a lignocellulose degradation system constituted of a two-phase process, and FIG. 1(c) is a diagram illustrating a lignocellulose degradation system constituted of a single-phase process.

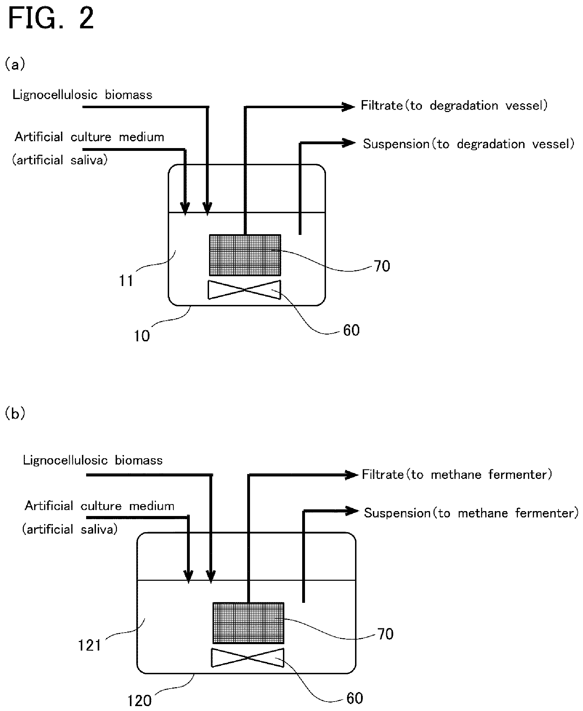

FIG. 2 is a diagram illustrating an example of a vessel constituting a lignocellulose degradation system for implementing a lignocellulose degradation method according to the present embodiment. FIG. 2(a) is a diagram illustrating an example of a culture vessel constituting the three-phase lignocellulose degradation system illustrated in FIG. 1(a). FIG. 2(b) is a diagram illustrating an example of a culture and degradation vessel constituting the two-phase lignocellulose degradation system illustrated in FIG. 1(b).

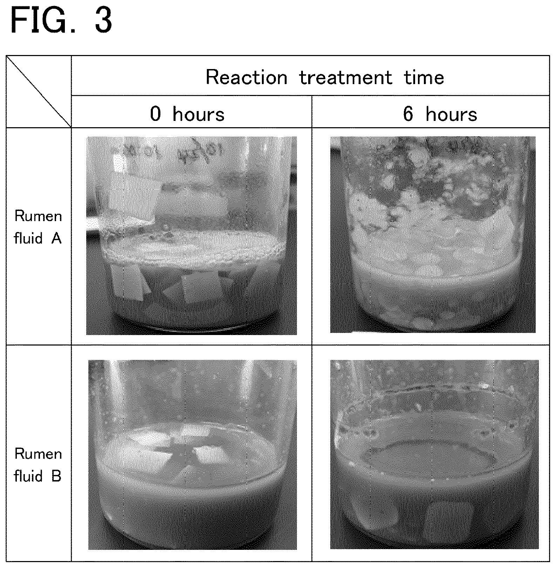

FIG. 3 is a diagram showing evaluation tests of lignocellulose degradation by rumen fluid A and rumen fluid B in Examples.

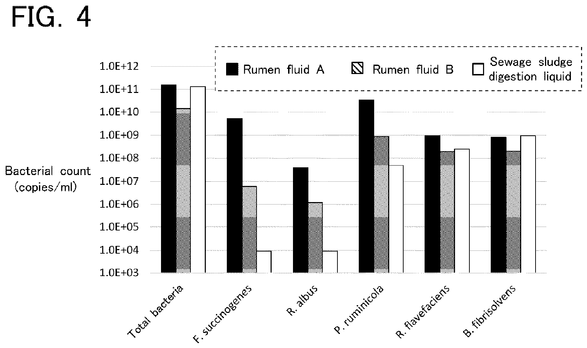

FIG. 4 is a graph showing the numbers of total bacteria and five types of lignocellulolytic bacteria contained in rumen fluid A, rumen fluid B, and a sewage sludge digestion liquid in Examples.

### Description of Embodiments

To clarify the contribution of bacteria (rumen microorganisms) living in rumen fluid present in the paunch of ruminants to lignocellulose degradation, the present inventors quantified bacterial counts by a quantitative PCR method. The relationship between the bacterial counts of representative five types of lignocellulolytic bacteria living in rumen fluid and lignocellulolytic activity was investigated, and the results revealed that there are approximately positive proportional relationships between the abundances of three types of bacteria, *Fibrobacter succinogenes, Ruminococcus albus,* and *Prevotella* and lignocellulolytic activity of rumen fluid. This led to a finding that lignocellulosic biomass can be efficiently degraded by selecting a rumen fluid with high lignocellulolytic activity in view of abundances of the three types of bacteria or by evaluating lignocellulolytic activity while monitoring culture of rumen microorganisms using the number of a specific bacterium as an indicator.

Embodiments and drawings related to a lignocellulose degradation system and a method for degrading lignocellulose according to the present invention will be specifically described below. However, the present invention is not intended to be limited to the configurations described in the embodiments and drawings explained below.

### Configurations of lignocellulose degradation system

Three types of lignocellulose degradation systems employing the method for degrading lignocellulose according to the present embodiment will be described with reference to FIG. 1.

FIG. 1(a) illustrates a lignocellulose degradation system 1 constituted of a three-phase process. In addition, FIG. 2(a) is a diagram illustrating an example of a culture vessel 10 constituting the lignocellulose degradation system 1 illustrated in FIG. 1(a). As illustrated in FIG. 1(a), the lignocellulose degradation system 1 includes a culture vessel 10 for performing culture of rumen microorganisms, a degradation vessel 20 in which the cultured rumen microorganisms are used to convert (degrade) lignocellulosic biomass into a form that can be easily utilized in a subsequent methane fermenter 30, and the methane fermenter 30. Upon supplying to the culture vessel 10 a rumen fluid collected from cattle or the like, an artificial culture medium (artificial saliva), and lignocellulosic biomass crushed and/or ground by a grinder 40 or the like as necessary, lignocellulolytic bacteria present in the rumen fluid degrade and metabolize lignocellulose to proliferate as well as produce lignocellulose degradation products (partial degradation products of lignocellulose and volatile fatty acids (VFAs), such as acetic acid). As illustrated in FIG. 2(a), in the culture vessel 10, a culture fluid 11 is stirred by a stirrer 60, and a liquid (filtrate) subjected to solid-liquid separation through a membrane 70 (filter) or the like and a suspension containing undegraded or partially degraded lignocellulosic biomass are each sent to a downstream degradation vessel 20 (also referred to as a pretreatment vessel). The system can control hydraulic retention time (HRT) and solids retention time (SRT) of the culture fluid 11 in the culture vessel 10 by appropriately adjusting the amounts of the filtrate and the suspension of the culture fluid 11 withdrawn from the culture vessel 10 and sent to the degradation vessel 20. The filtrate and the suspension of the culture fluid 11 contain many lignocellulolytic bacteria. Thus, upon supplying lignocellulosic biomass to the degradation vessel 20, the lignocellulolytic reaction proceeds under the action of lignocellulolytic bacteria and produces a reaction liquid containing volatile fatty acids (VFAs), such as acetic acid, and partial degradation products of lignocellulose. Upon supplying the reaction liquid containing a large amount of lignocellulose degradation products from the degradation vessel 20 to the methane fermenter 30, methane fermentation using the lignocellulose degradation products as substrates (raw materials) proceeds in a methane fermentation liquid contained in the methane fermenter 30, and methane is produced. Thereafter, a methane fermentation residue in the methane fermenter 30 is sent to a dehydrator 50. A dehydration-separated liquid discharged from the dehydrator 50 contains materials serving as a nitrogen source and/or a phosphorus source and thus may be refluxed and added to the culture vessel 10. In addition, concentrated sludge discharged from the dehydrator 50 may be used as a fertilizer, a construction material, or the like. The lignocellulose degradation system 1 constituted of the three-phase process illustrated in FIG. 1(a) has functions divided into steps of (1) culture of rumen microorganisms, (2) degradation (pretreatment) of lignocellulose, and (3) methane fermentation, thus providing an advantage of facilitating the operational control.

FIG. 1(b) illustrates a lignocellulose degradation system 101 constituted of a two-phase process. In addition, FIG. 2(b) is a diagram illustrating an example of a culture and degradation vessel 120 constituting the lignocellulose degradation system 101 illustrated in FIG. 1(b). As illustrated in FIG. 1(b), the lignocellulose degradation system 101 includes a culture and degradation vessel 120 for performing both culture of rumen microorganisms and degradation (pretreatment) of lignocellulosic biomass, and the methane fermenter 130. Upon supplying to the culture and degradation vessel 120 a rumen fluid collected from cattle or the like, an artificial culture medium (artificial saliva), and lignocellulosic biomass crushed and/or ground by a grinder 40 or the like as necessary, lignocellulolytic bacteria present in the rumen fluid degrade and metabolize lignocellulose to proliferate as well as lignocellulolytic reaction proceeds under the action of lignocellulolytic bacteria and produces a reaction liquid containing volatile fatty acids (VFAs), such as acetic acid, and partial degradation products of lignocellulose. As illustrated in FIG. 2(b), in the culture and degradation vessel 120, a reaction liquid 121 is stirred by a stirrer 60, and a liquid (filtrate) subjected to solid-liquid separation through a membrane 70 (filter) or the like and a suspension containing undegraded or partially degraded lignocellulosic biomass are each sent to a downstream methane fermenter 130. The system can control hydraulic retention time (HRT) and solids retention time (SRT) of the reaction liquid 121 in the culture and degradation vessel 120 by appropriately adjusting the amounts of the filtrate and the suspension of the reaction liquid 121 withdrawn from the culture and degradation vessel 120 and sent to the methane fermenter 130. Upon supplying the reaction liquid 121 containing lignocellulose degradation products to the methane fermenter 130, methane fermentation using the lignocellulose degradation products as substrates (raw materials) proceeds in a methane fermentation liquid contained in the methane fermenter 130, and methane is produced. Thereafter, a methane fermentation residue in the methane fermenter 130 is sent to a dehydrator 50. A dehydration-separated liquid discharged from the dehydrator 50 contains materials serving as a nitrogen source and/or a phosphorus source and thus may be refluxed and added to the culture and degradation vessel 120. In addition, concentrated sludge discharged from the dehydrator 50 may be used as a fertilizer, a construction material, or the like. The two-phase process illustrated in FIG. 1(b) is a process in which a culture step and a degradation step (pretreatment step) can be performed in one vessel (the culture and degradation vessel 120) and is known as a rumen derived anaerobic digestion (RUDAD) process (Non-Patent Literature: Huub J. Gijzen et al., Biotechnology and Bioengineering, Vol. 31, pp. 418-425 (1988)). The present process requires fewer vessels than the three-phase process and thus can be constructed at lower cost, and the equipment can be made compact.

FIG. 1(c) illustrates a lignocellulose degradation system 201 that does not have the culture vessel 10, the degradation vessel 20, and the culture and degradation vessel 120, and is constituted of a single-phase process in which a rumen fluid selected using the number of a specific bacterium as an indicator is directly added to a methane fermenter 230, and degradation of lignocellulosic biomass and methane fermentation are carried out in the methane fermenter 230. Upon supplying to the methane fermenter 230 a rumen fluid collected from cattle or the like and lignocellulosic biomass crushed and/or ground by a grinder 40 or the like as necessary, lignocellulolytic reaction proceeds under the action of lignocellulolytic bacteria present in the rumen fluid and produces a reaction liquid containing volatile fatty acids (VFAs), such as acetic acid, and partial degradation products of lignocellulose, and methane fermentation proceeds using these as substrates or using hydrogen and carbon dioxide as substrates. In the single-phase process, a selected rumen fluid with high lignocellulolytic activity is directly reacted with lignocellulosic biomass, and thus an artificial culture medium (artificial saliva) need not be added but may be appropriately added as necessary. The single-phase process does not have the culture vessel 10, the degradation vessel 20, and the like and thus can be constructed at the lowest cost, and the equipment can be made more compact.

### Method for degrading lignocellulose

The method for degrading lignocellulose in an embodiment of the present invention will be described in detail below. Lignocellulose, which is a main component of cell walls of plant cells, is composed of hemicellulose, cellulose, and lignin which are strongly bonded to each other. In rumen fluid present in the paunch (rumen) of ruminants, such as cattle, many rumen microorganisms that produce lignocellulose-degrading enzymes are present. The method for degrading lignocellulose in an embodiment of the present invention degrades lignocellulose by utilizing the action of rumen microorganisms with high lignocellulolytic activity and produces volatile fatty acids, such as acetic acid. Based on the positive proportional relationships between abundances of three types of bacteria, *Fibrobacter succinogenes, Ruminococcus albus,* and *Prevotella ruminicola*, among rumen microorganisms, and lignocellulolytic activity, a rumen fluid is selected using as an indicator the number of at least one type of bacteria of these three types of bacteria, and the selected rumen fluid is used. In addition, lignocellulolytic activity is evaluated while culture of rumen microorganisms is monitored. With these two points, the method can degrade lignocellulosic biomass stably and efficiently.

Examples of lignocellulosic biomass used as a raw material include unutilized agricultural and forestry wastes, such as timber from forest thinning, rice straws, chaff, bagasse, and cogon grass; as well as lignocellulosic industrial wastes, such as vegetable scraps, used tea leaves, coffee grounds, bean curd refuse, shochu lees, construction waste materials, used paper and waste paper, and municipal wastes; or biomass resource crops, such as *Erianthus* and giant miscanthus. In addition, paper shredded by a shredder is difficult to recycle as the fibers are broken and thus is incinerated, but such shredded paper can also be used as a raw material. When lignocellulose degradation of lignocellulosic biomass proceeds and volatile fatty acids, such as acetic acid, are produced, they can be used as substrates (raw materials) for methane fermentation, and thus methane can be efficiently produced. Furthermore, even when fermentation does not proceed to produce volatile fatty acids but when lignocellulose is degraded to a form with a low molecular weight, such as cellobiose, or degraded to a constituent sugar, such as glucose, these can be used as substrates (raw materials) for methane fermentation, and thus methane can be efficiently produced.

### Step of selecting rumen fluid

Rumen fluid is a digestive juice present in the paunch (rumen) of ruminants. Examples of ruminants include cattle, sheep, goats, deer, camels, and llamas. For example, the paunch of adult cattle has a volume of 150 to 200 L, and many lignocellulolytic bacteria, hemicellulolytic bacteria, ligninolytic bacteria, amylolytic bacteria, methanogenic bacteria, and the like live in rumen fluid. Lignocellulolytic bacteria can produce enzymes that degrade lignocellulose (fiber), such as cellulase. Thus, when fiber, such as grass, is ingested by ruminants, lignocellulolytic bacteria degrade lignocellulose and produce volatile fatty acids (VFAs), such as acetic acid, propionic acid, butyric acid, and valeric acid, which serve as energy sources for ruminants. The methanogenic bacteria contained in rumen fluid can produce methane using acetic acid produced by lignocellulolytic bacteria as a substrate or using hydrogen and carbon dioxide as substrates. A rumen fluid collected from the paunch of a ruminant using a tube, such as a catheter, may be used, or a rumen fluid generated from a meat processing plant or the like may be used.

The rumen fluid can be selected using as an indicator the number of at least one type of bacteria of three types of lignocellulolytic bacteria, *Fibrobacter succinogenes, Ruminococcus albus,* and *Prevotella ruminicola.* The quantification of the numbers of these bacteria is not particularly limited but is preferably performed by a quantitative PCR method, which can rapidly and accurately measure the numbers of specific bacteria.

When a quantitative PCR method is used to quantify the numbers of lignocellulolytic bacteria, genomic DNA extracted from a rumen fluid and purified is used. Strains of lignocellulolytic bacteria are identified by gene sequence analysis of 16S rRNA. Thus, the number of target bacteria can be quantified based on the number of amplifications (copies) of the gene region of the target gene 16S rRNA using a PCR primer or a probe.

### Step of culturing rumen microorganisms

For the degradation of lignocellulosic biomass, a rumen fluid obtained from a ruminant may be used, or a culture fluid of rumen microorganisms may be used. The culture fluid of rumen microorganisms may be obtained by adding a concentrated liquid of a culture fluid to a rumen fluid and culturing the mixture, or may be obtained by adding a liquid culture medium to a precipitate (bacterial cells) obtained by centrifuging a rumen fluid and culturing the mixture. In culture of rumen microorganisms, a culture fluid of rumen microorganisms with high lignocellulolytic activity can be obtained by adjusting temperature, oxidation-reduction potential (ORP), hydraulic retention time (HRT) or solids retention time (SRT), and ammonium nitrogen concentration in a culture medium using as an indicator abundance of at least one or more types of bacteria of three types, *Fibrobacter succinogenes, Ruminococcus albus,* and *Prevotella ruminicola.* The quantification of the numbers of these bacteria is not particularly limited but is preferably performed by a quantitative PCR method, which can rapidly and accurately measure the numbers of specific bacteria. When a quantitative PCR method is used to quantify the numbers of lignocellulolytic bacteria, genomic DNA extracted from a culture fluid and purified is used. A culture fluid of rumen microorganisms can be cultured in a large quantity or can also be subcultured, and thus a culture fluid of rumen microorganisms obtained by adjusting the numbers of the bacteria can be used for lignocellulose degradation as necessary.

The culture medium used for the culture fluid of rumen microorganisms may be a natural culture medium whose base material originates from a natural product; or a synthetic culture medium in which various nutrients necessary for the growth of rumen microorganisms are all composed of chemical agents. In particular, the culture medium preferably contains a carbon source, such as cellulose or hemicellulose, in addition to a nitrogen source such as an ammonium salt, a phosphorus source such as a phosphate salt, and the like, which are useful nutrient sources for rumen microorganisms. In addition, as illustrated in FIGS. 1(a) and 1(b), the dehydration-separated liquid discharged from the dehydrator 50 contains materials serving as a nitrogen source and/or a phosphorus source and thus may be added as a nutrient source to the culture medium. Furthermore, upon production of volatile fatty acids (VFAs), such as acetic acid, in a degradation step after the culture step, the pH in the reaction liquid decreases, and/or the osmotic pressure increases, and this may change abundances and functions of lignocellulolytic bacteria. Thus, a buffering agent is preferably added to the culture medium to mitigate the change in pH and/or the increase in osmotic pressure. Ruminant saliva has a high buffering capacity, and thus an artificial saliva that simulates ruminant saliva is preferably used as a buffering agent. Examples of the buffering agent include sodium chloride, sodium bicarbonate, phosphate salts, and potassium chloride. The pH of the culture medium is adjusted to from 6.0 to 7.5 and preferably from 6.5 to 7.0 by adding an acid or an alkali as necessary. Moreover, water used for the culture medium is preferably tap water or groundwater. Still more, the ammonium nitrogen concentration in the culture medium is controlled by adding water or supplying a dehydration-separated liquid to give a concentration of 50 to 2000 mg/L and more preferably of 60 to 500 mg/L.

In the culture vessel 10 illustrated in FIG. 1(a) or the culture and degradation vessel 120 illustrated in FIG. 1(b), to make the step of culturing rumen microorganisms anaerobic to simulate the environment in the rumen, the oxidation-reduction potential of the culture fluid of rumen microorganisms is adjusted to -100 mv or less, more preferably to -200 mv or less, and even more preferably to approximately -250 mv. Rumen microorganisms (lignocellulolytic bacteria) are anaerobic bacteria. Thus, when the ORP of the culture medium becomes higher than a predetermined ORP (when the state becomes close to an aerobic state), an action may be taken, including injecting an inert gas, such as nitrogen gas or carbon dioxide gas; adding a reducing substance, such as cysteine, L-ascorbic acid, sodium sulfide, ascorbic acid, methionine, thioglycol, or DTT; or adding an organic substance to consume oxygen by the action of facultative anaerobes. Alternatively, the culture step may be performed in a closed system under a nitrogen or carbon dioxide atmosphere.

Collecting and transporting a rumen fluid for use every time a process for lignocellulosic biomass degradation is performed involves a great deal of cost and energy consumption. Thus, rumen microorganisms (lignocellulolytic bacteria) are desirably cultured stably over a long period of time to reduce the collection frequency of the rumen fluid. For the culture of ruminal microorganisms in the culture vessel 10 or the culture and degradation vessel 120, it is considered important that the solids retention time (SRT) is longer than the hydraulic retention time (HRT). That is, lignocellulolytic bacteria adhere to the surface of solid matter (lignocellulosic biomass) and proliferate, and thus, if the SRT is short, they would be discharged out of the system together with the solid matter. On the other hand, an HRT longer than necessary would be a negative factor, such as causing a decrease in pH due to produced volatile fatty acids (VFAs) or inhibiting growth of the microorganisms. Specifically, the HRT is adjusted to from 8 hours to 36 hours and preferably from 10 hours to 24 hours, and the SRT is adjusted to 24 hours or longer and preferably from 48 hours to 72 hours. As illustrated in FIGS. 2(a) and 2(b), the HRT and the SRT cannot be individually adjusted in a vessel of a complete mixing system, in which the culture fluid is stirred, that is, the HRT and the SRT become equal. Thus, the filtrate and the suspension are individually discharged from the culture vessel 10 or the culture and degradation vessel 120 by a method, such as utilizing the membrane 70 (filter). In addition, the temperature of the reaction system in the culture step is controlled to from 35 to 42°C and more preferably from 37 to 40°C utilizing a temperature sensor or the like. The solid concentration in lignocellulosic biomass added to the culture vessel 10 or the culture and degradation vessel 120 is from 0.05 to 20 wt.% and more preferably from 0.1 to 5 wt.%.

### Degradation step (pretreatment step)

In a degradation step (also referred to as a pretreatment step), lignin in lignocellulosic biomass is partially degraded by ligninolytic enzymes produced by lignocellulolytic bacteria, loosening the strong structure of lignocellulose, then the lignocellulosic biomass is degraded into cellulose by endoglucanase and exoglucanase or into hemicellulose by xylanase or the like, and they are converted into hexoses (hexasaccharides), such as glucose. Furthermore, pyruvic acid or the like is produced from hexoses (hexasaccharide), such as glucose, and when the reaction further proceeds, volatile fatty acids (VFAs), such as acetic acid, propionic acid, butyric acid, and formic acid, are produced. In addition, hydrogen and carbon dioxide are also produced by metabolic processes. Acetic acid, hydrogen, and carbon dioxide are used as substrates (raw materials) for methane fermentation. Thus, when the production amounts of acetic acid, hydrogen, and carbon dioxide increase with the promotion of degradation of lignocellulose, the production amount of methane also increases. In the degradation step (pretreatment step), lignocellulose need not be degraded and/or metabolized to hexoses (hexasaccharides) or volatile fatty acids (VFAs); it is sufficient for lignocellulose, which is difficult to degrade, to be degraded to a form easily utilized in a subsequent methane fermentation step, for example, to a form as small as oligosaccharides. In the degradation step (pretreatment step), lignocellulosic biomass contained in the reaction liquid is from 0.5 to 30 wt.% and more preferably from 0.5 to 10 wt.%.

To approximate the degradation step to the environment in the rumen of ruminants and improve the efficiency of lignocellulose degradation in the degradation vessel 20 illustrated in FIG. 1(a) or the culture and degradation vessel 120 illustrated in FIG. 1(b), the degradation of lignocellulosic biomass is preferably controlled based on a parameter including temperature, pH, oxidation-reduction potential (ORP), hydraulic retention time (HRT) or solids retention time (SRT), and ammonium nitrogen concentration in the culture medium, and preferably monitored using the number of bacteria in the culture fluid or reaction liquid as an indicator. The quantification of the numbers of these bacteria is not particularly limited but is preferably performed by a quantitative PCR method, which can rapidly and accurately measure the numbers of specific bacteria. When a quantitative PCR method is used to quantify the numbers of lignocellulolytic bacteria, genomic DNA extracted from the culture fluid 11 or the reaction liquid 121 and purified is used.

To increase the activity of lignocellulolytic bacteria present in the rumen fluid, an ammonium salt as a nitrogen source, a phosphate salt as a phosphorus source, and/or the like, which serves as a nutrient source for lignocellulolytic bacteria, may be appropriately added to the culture medium. As illustrated in FIG. 1(b), the dehydration-separated liquid discharged from the dehydrator contains materials serving as a nitrogen source and/or a phosphorus source and thus may be added to the culture medium. In addition, upon production of volatile fatty acids (VFAs), such as acetic acid, in the degradation step, the pH in the reaction liquid decreases, and/or the osmotic pressure increases, and this may change abundances and functions of cellulolytic bacteria. Thus, a buffering agent is preferably added to the culture medium to mitigate the change in pH and/or the increase in osmotic pressure. Ruminant saliva has a high buffering capacity, and thus an artificial saliva that simulates ruminant saliva is preferably used as a buffering agent. The pH of the culture medium is adjusted to from 6.0 to 7.5 and preferably from 6.5 to 7.0 by adding an acid or an alkali as necessary. Water used for the culture medium or dilution of the reaction liquid is preferably tap water or groundwater. Furthermore, the ammonium nitrogen concentration in the culture medium is controlled by adding water or supplying a dehydration-separated liquid to give a concentration of 50 to 2000 mg/L and more preferably of 60 to 500 mg/L.

In the degradation vessel 20 or the culture and degradation vessel 120, to make the degradation step anaerobic to simulate the environment in the rumen of ruminants, the oxidation-reduction potential of the reaction liquid is adjusted to -100 mv or less, more preferably to -200 mv or less, and even more preferably to approximately -250 mv. Rumen microorganisms (lignocellulolytic bacteria) are anaerobic bacteria. Thus, when the ORP of the culture medium becomes higher than a predetermined ORP (when the state becomes close to an aerobic state), an action is taken, including injecting an inert gas, such as nitrogen gas or carbon dioxide gas; adding a reducing substance, such as cysteine, L-ascorbic acid, sodium sulfide, ascorbic acid, methionine, thioglycol, or DTT; or adding an organic substance to consume oxygen by the action of facultative anaerobes. The degradation step may be performed in a closed system under a nitrogen or carbon dioxide atmosphere.

The reaction time of lignocellulosic biomass and lignocellulolytic bacteria in the degradation step in the degradation vessel 20 illustrated in FIG. 1(a) or the culture and degradation vessel 120 illustrated in FIG. 1(b) is desirably controlled by the hydraulic retention time (HRT) or solids retention time (SRT). The HRT is controlled to from 8 to 36 hours and preferably from 10 to 24 hours, and the SRT is controlled to 24 hours or longer and preferably from 48 to 72 hours. As illustrated in FIG. 2(b), the HRT and the SRT cannot be individually adjusted in a complete mixing system, in which the reaction liquid is stirred, that is, the HRT and the SRT become equal. Thus, the membrane 70 (filter) membrane is provided in the culture and degradation vessel 120, or stirring in these vessels is gently performed to prevent complete mixing. And the HRT and the SRT are controlled by appropriately adjusting the amount of the membrane filtrate from which solid matter is removed and/or the amount of the suspension containing a supernatant and solid matter removed out of the vessel. For the degradation vessel 20 illustrated in FIG. 1(a), the HRT and the SRT are individually controlled in the upstream culture vessel 10. Thus, the HRT and the SRT need not be individually controlled in the degradation vessel 20, and the degradation may be carried out in a complete mixing system. In this case, the reaction liquid in the degradation vessel 20 is not separated into the filtrate and the suspension, the HRT and the SRT become equal. Both the HRT and the SRT in the degradation vessel 20 are preferably from 4 to 36 hours. Furthermore, the temperature of the reaction system in the degradation step is controlled to from 35 to 42°C and more preferably from 37 to 40°C utilizing a temperature sensor or the like. The reaction in the degradation step may be carried out stationarily or under stirring but is preferably carried out under stirring to accelerate the progress of the degradation step.

The culture step and the degradation step may be performed independently in the culture vessel 10 and in the degradation vessel 20, respectively, as illustrated in FIG. 1(a) or may be performed in one vessel, in the culture and degradation vessel 120, as illustrated in FIG. 1(b).

### Methane fermentation step

Lignocellulolytic bacteria and methanogenic bacteria in rumen fluid are closely related to each other, and the activity of methanogenic bacteria is theoretically predicted to increase when the activity of lignocellulolytic bacteria increases. The method allows the methane fermentation to proceed stably and efficiently and can improve the production of methane by selecting a rumen liquid using as an indicator the number of at least one type of bacteria of three types of lignocellulolytic bacteria, *Fibrobacter succinogenes, Ruminococcus albus,* and *Prevotella* and using the selected rumen fluid; and by evaluating the degradation treatment in the degradation step by monitoring the culture step using the numbers of the specific bacteria as indicators.

In the methane fermentation step, methane fermentation is carried out using the following as substrates: lignocellulosic biomass in which lignocellulose, which is difficult to degrade, is degraded to a form easily utilized in the methane fermentation step, for example, to a form as small as oligosaccharides; or volatile fatty acids (lignocellulose degradation products), such as acetic acid, produced in the degradation step (pretreatment step), hydrogen, and carbon dioxide. The methane fermentation is carried out by methanogenic bacteria under anaerobic conditions, which are equivalent to those of the degradation step by lignocellulolytic bacteria.

The methane fermentation may be either wet methane fermentation or dry methane fermentation. In addition, the methane fermentation may be either medium temperature methane fermentation or high temperature methane fermentation. However, when all of the lignocellulolytic bacteria culture step, the lignocellulose degradation step, and the methane fermentation step are carried out in one vessel, medium temperature methane fermentation is preferred. The medium temperature fermentation is preferably performed for 20 to 30 days, more preferably for 23 to 27 days, and even more preferably for approximately 25 days, and the high temperature fermentation is preferably performed for 10 to 20 days, more preferably for 13 to 17 days, and even more preferably for approximately 15 days. Furthermore, the temperature of the methane fermentation by medium temperature fermentation is higher than 20°C and 40°C or lower, more preferably 30°C or higher and 37°C or lower, and even more preferably approximately 37°C, and the temperature of the methane fermentation by high temperature fermentation is higher than 40°C and 60°C, more preferably from 50 to 55°C, and even more preferably approximately 55°C.

The methane fermentation step may be carried out in the methane fermenter 30 or 130 as illustrated in FIGS. 1(a) and 1(b) using as substrates lignocellulose degradation products contained in the reaction liquid sent from the pretreatment vessel (degradation vessel 20 or culture and degradation vessel 120). Alternatively, the methane fermentation may be carried out by adding the selected rumen fluid and lignocellulosic biomass to the methane fermenter 230 as illustrated in FIG. 1(c).

### Examples

An "evaluation test of lignocellulolytic activity" and "quantification of abundances of total bacteria and lignocellulolytic bacteria" were carried out for rumen liquids and a sewage sludge digestion liquid with different abundances of rumen microorganisms.

### Evaluation test of lignocellulolytic activity

### Test Example 1

Into a 150-mL volume borosilicate glass vial, 1.5 g of copy paper cut into about 1-cm square pieces and 15 mg of cysteine hydrochloride monohydrate were added, and 30 mL of rumen liquid A from which solid matter was removed with a 1-mm mesh sieve was mixed. The vial was filled with nitrogen gas to create an anaerobic state, then sealed and placed in a shaking incubator, and rotary-shaken at 130 rpm at 39°C. After 6 hours, the state of degradation (dissolution) of the copy paper was visually observed.

### Test Example 2

The test was performed all in the same manner as in Test Example 1 except that rumen fluid B was used.

### Test Example 3

The test was performed all in the same manner as in Test Example 1 except that an anaerobic digestion sludge of a food factory was used instead of the rumen fluid.

FIG. 3 is a diagram showing evaluation tests of lignocellulose degradation by rumen fluid A and rumen fluid B. For the copy paper treated with rumen solution A, the reaction liquid became pasty to the extent that the original shape of the copy paper did not remain 6 hours after the treatment, confirming that lignocellulose degradation proceeded. The copy paper treated with rumen solution B swelled and the corners were rounded 6 hours after the treatment, confirming that lignocellulose degradation proceeded slightly. On the other hand, for the copy paper treated with the anaerobic digestion sludge of a food factory, no change was observed even 6 hours after the treatment, confirming that lignocellulose degradation did not proceed (not shown). The above test results revealed that rumen fluid A had the highest lignocellulolytic activity, rumen fluid B had the second highest lignocellulolytic activity, and the anaerobic digestion sludge of a food factory showed no lignocellulolytic activity.

### Quantification of abundances of total bacteria and lignocellulolytic bacteria

Abundances of total bacteria and five types of major lignocellulolytic bacteria *(Fibrobacter succinogenes, Ruminococcus albus, Prevotella ruminicola*, *Ruminococcus flavefaciens,* and *Butyrivibrio fibrisolvens*) living in rumen fluid A, rumen fluid B, and a sewage sludge digestion liquid with different lignocellulolytic activities were quantified by a quantitative PCR method. Table 1 shows primers/probes and base sequences used for the quantitative PCR, and Table 2 shows the conditions for the quantitative PCR.

**[Table 1]**

| Bacterial name | Target gene | SEQ ID NO | Primer/probe sequence (5' → 3') | Literature |
|---|---|---|---|---|
| Total bacteria (eubacteria) | 16S rRNA | 1 | F: CGGTGAATACGTTCYCGG | 1 |
| | | 2 | R: GGWTACCTTGTTACGACTT | |
| | | 3 | P: FAM-CTTGTACACACCGCCCGTC- TAMRA | |
| *Fibrobacter succinogenes* | 16S rRNA | 4 | F: GTTCGGAATTACTGGGCGTAAA | 2 |
| | | 5 | R: CGCCTGCCCCTGAACTATC | |
| *Ruminococcus albus* | 16S rRNA | 6 | F: CCCTAAAAGCAGTCTTAGTTCG | 3 |
| | | 7 | R: CCTCCTTGCGGTTAGAACA | |
| *Prevotella ruminicola* | 16S rRNA | 8 | F: ATGGATGCCCGCTGTTTG | 4 |
| | | 9 | R: ATGTTCCTCCGCTTGTGC | |
| *Ruminococcus flavefaciens* | 16S rRNA | 10 | F: GCCGAAGGTGGGATTGATGA | 5 |
| | | 11 | R: GAAAGGAGGTGATCCAGCCG | |
| *Butyrivibrio fibrosolvens* | 16S rRNA | 12 | F: ACACACCGCCCGTCACA | 6 |
| | | 13 | R: TCCTTACGGTTGGGTCACAGA | |

| | | | | |
|---|---|---|---|---|
| F: forward primer / R: reverse primer / P: probe Literatures 1. Suzuki, M. T., Taylor, L. T., and Delong, E. F. Appl. Environ. Microbiol. 2000, 66, 4605-4614. 2. Denman, S. E., McSweeney, C. S. FEMS Microbiol. Ecol. 2006, 58, 572-582. 3. Khafipour, E., Li, S., Plaizier, J. C., and Krause, D. O. Appl. Environ. Microbiol. 2009, 75, 7115-7124. 4. Wang, X., Li, X., Zhao, C., Hu, P., Chen, H., Liu, Z., Liu, G., and Wang, Z. Appl. Environ. Microbiol. 2012, 78, 2386-2392. 5. Lukić, I., Getselter, D., Ziv, O., Reuveni, E., Koren, O., and Elliot, E. Transl. Psychiatry 2019, 9, 133. 6. Hussain, S.K.A., Srivastava, A., Tyagi, A., Shandilya, U.K., Kumar, A., Kumar, S., Panwar, S., and Tyagi, A. K. 3 Biotech. 2016, 6, 90. 6, 90. | | | | |

**[Table 2]**

| Bacterial name | Primer/probe | PCR reaction conditions | | | |
|---|---|---|---|---|---|
| | Concentration (nM) | Step | Reaction temperature (°C) | Time | Cycle number |
| Total bacteria (eubacteria) | F: 200 | 1 | 50 | 2min | 1 |
| | R: 300 | 2 | 95 | 10 min | 1 |
| | | | 95 | 15 sec | 35 |
| | P: 200 | 3 | 56 | 1 min | |
| *Fibrobacter succinogenes* | | 1 | 95 | 2 min | 1 |
| | | 2 | 95 | 15 sec | 40 |
| | F: 300 | | 60 | 1 min | |
| | R: 300 | | 95 | 5 sec | |
| | | Dissociation | 65 | 60 sec | 1 |
| | | | 95 | - | |
| *Ruminococcus albus* | | 1 | 95 | 2 min | 1 |
| | | 2 | 95 | 15 sec | 40 |
| | F: 500 | | 60 | 1 min | |
| | R: 500 | | 95 | 5 sec | |
| | | Dissociation | 65 | 60 sec | 1 |
| | | | 95 | - | |
| *Prevotella ruminicola* | | 1 | 95 | 2 min | 1 |
| | | 2 | 95 | 15 sec | 40 |
| | F: 500 | | 60 | 1 min | |
| | R: 500 | | 95 | 5 sec | |
| | | Dissociation | 65 | 60 sec | 1 |
| | | | 95 | - | |
| *Ruminococcus flavefaciens* | | 1 | 95 | 2 min | 1 |
| | | 2 | 95 | 15 sec | 40 |
| | F: 500 | | 60 | 1 min | |
| | R: 500 | | 95 | 5 sec | |
| | | Dissociation | 65 | 60 sec | 1 |
| | | | 95 | - | |
| *Butyrivibrio fibrosolvens* | F: 500 | 1 | 95 | 10 min | 1 |
| | R: 500 | | 95 | 30 sec | |
| | | 2 | 55 | 45 sec | 40 |
| | | | 72 | 30 sec | |
| | | | 95 | 5 sec | |
| | | Dissociation | 65 | 60 sec | 1 |
| | | | 95 | - | |

### Extraction of DNA

DNA was extracted from each of rumen liquids A and B, and the sewage sludge digestion liquid and purified. Using this DNA as a substrate, abundances of total bacteria and the five types of lignocellulolytic bacteria were measured by a quantitative PCR method using a PCR primer and/or a probe shown in Tables 1 and 2.

### Quantification of total bacterial count

The total bacteria (eubacteria) were quantified by the TaqMan (trade name) Probe method using the forward primer (F) of SEQ ID NO: 1, the reverse primer (R) of SEQ ID NO: 2, and the probe (P) of SEQ ID NO: 3 shown in Table 1. A PCR reaction was performed under the conditions shown in Table 2 using Eagel Taq Universal Master Mix (ROX) (available from Roche Diagnostics K.K.) as a quantitative PCR reagent, and the numbers of total bacteria present in rumen fluids A and B, and the sewage sludge digestion liquid were quantified based on the number of amplifications (copies) of the gene region of the target gene 16S rRNA.

### Quantification of Fibrobacter succinogenes

Quantification was performed by the SYBR (trade name) Green method using the forward primer (F) of SEQ ID NO: 4 and the reverse primer (R) of SEQ ID NO: 5 shown in Table 1. A PCR reaction was performed under the conditions shown in Table 2 using Power Up SYBR (trade name) Green Master Mix (available from Thermo Fisher Scientific Inc.) as a quantitative PCR reagent, and the numbers of bacteria present in rumen fluids A and B, and the sewage sludge digestion liquid were quantified based on the number of amplifications (copies) of the gene region of the target gene 16S rRNA.

### Quantification of Ruminococcus albus

Quantification was performed by the SYBR (trade name) Green method using the forward primer (F) of SEQ ID NO: 6 and the reverse primer (R) of SEQ ID NO: 7 shown in Table 1. A PCR reaction was performed under the conditions shown in Table 2 using Power Up SYBR (trade name) Green Master Mix (available from Thermo Fisher Scientific Inc.) as a quantitative PCR reagent, and the numbers of bacteria present in rumen fluids A and B, and the sewage sludge digestion liquid were quantified based on the number of amplifications (copies) of the gene region of the target gene 16S rRNA.

### Quantification of Prevotella ruminicola

Quantification was performed by the SYBR (trade name) Green method using the forward primer (F) of SEQ ID NO: 8 and the reverse primer (R) of SEQ ID NO: 9 shown in Table 1. A PCR reaction was performed under the conditions shown in Table 2 using THUNDERBIRD (trade name) SYBR (trade name) qPCR Mix (available from Toyobo Co., Ltd.) as a quantitative PCR reagent, and the numbers of bacteria present in rumen fluids A and B, and the sewage sludge digestion liquid were quantified based on the number of amplifications (copies) of the gene region of the target gene 16S rRNA.

### Quantification of Ruminococcus flavefaciens

Quantification was performed by the SYBR (trade name) Green method using the forward primer (F) of SEQ ID NO: 10 and the reverse primer (R) of SEQ ID NO: 11 shown in Table 1. A PCR reaction was performed under the conditions shown in Table 2 using Power Up SYBR (trade name) Green Master Mix (available from Thermo Fisher Scientific Inc.) as a quantitative PCR reagent, and the numbers of bacteria present in rumen fluids A and B, and the sewage sludge digestion liquid were quantified based on the number of amplifications (copies) of the gene region of the target gene 16S rRNA.

### Quantification of Butyrivibrio fibrisolvens

Quantification was performed by the SYBR (trade name) Green method using the forward primer (F) of SEQ ID NO: 12 and the reverse primer (R) of SEQ ID NO: 13 shown in Table 1. A PCR reaction was performed under the conditions shown in Table 2 using Gene Ace SYBR (trade name) qPCR Mix α No ROX (available from Nippon Gene Co., Ltd.) as a quantitative PCR reagent, and the numbers of bacteria present in rumen fluids A and B, and the sewage sludge digestion liquid were quantified based on the number of amplifications (copies) of the gene region of the target gene 16S rRNA.

FIG. 4 shows abundances, in 1 mL of each sample, of total bacteria and the five types of lignocellulolytic bacteria living in rumen fluid A, rumen fluid B, and the sewage sludge digestion liquid.

In the evaluation test of lignocellulolytic activity, three types of bacteria, *Fibrobacter succinogenes, Ruminococcus albus,* and *Prevotella ruminicola*, were present in the largest numbers in rumen fluid A, which had high lignocellulolytic activity, and were present in the smallest numbers in the sewage sludge digestion liquid, which showed almost no lignocellulolytic activity. In rumen liquor B, which had the second highest lignocellulolytic activity, the abundances of the three types of bacteria were intermediate between those in rumen liquor A and those in the sewage sludge digestion liquid. The degradation activity for lignocellulose (copy paper) was higher in rumen fluid A than in rumen fluid B, and this revealed that there are approximately positive proportional relationships between the numbers of these three types of bacteria and lignocellulolytic activity. On the other hand, the abundances of *Ruminococcus flavefaciens* and *Butyrivibrio fibrisolvens,* which were bacteria other than the above three types of bacteria, were almost the same in rumen fluid A, rumen fluid B, and the sewage sludge digestion liquid regardless of lignocellulolytic activity.

The abundances of the three types of bacteria present in rumen fluid A, which had high lignocellulolytic activity, were 5.44 × 10⁹ copies/mL for *Fibrobacter succinogenes,* 3.73 × 10⁷ copies/mL for *Ruminococcus albus,* and 3.44 × 10¹⁰ copies/mL for *Prevotella ruminicola.* This leads to the assumption that a rumen fluid with high lignocellulolytic activity can be obtained by selecting a rumen fluid using at least one or more of the following as an indicator: abundance in a rumen fluid is 1.0 × 10⁹ copies/mL or more for *Fibrobacter succinogenes,* 1.0 × 10⁷ copies/mL or more for *Ruminococcus albus,* or 1.0 × 10¹⁰ copies/mL or more for *Prevotella ruminicola.*

Thus, this revealed that a rumen fluid with high lignocellulolytic activity can be selected using as an indicator the abundance of at least one or more types of bacteria of three types of bacteria, *Fibrobacter succinogenes, Ruminococcus albus,* and *Prevotella ruminicola.* In addition, based on this, it is also assumed that using as an indicator the abundance of at least one or more types of bacteria of the three types of bacteria enables the evaluation of lignocellulolytic activity while culture of rumen microorganisms is monitored.

### Industrial Applicability

The lignocellulose degradation system and the method for degrading lignocellulose of the present invention can be utilized in applications in which an industrial waste, such as municipal waste like used paper or waste paper, and a lignocellulosic industrial waste, such as an agricultural or forestry waste, or a construction waste material, is subjected to degradation treatment to produce methane using the degraded product as a raw material.

### Reference Signs List

1, 101, 201 Lignocellulose degradation system
10 Culture vessel
11 Culture fluid
20 Degradation vessel
30, 130, 230 Methane fermenter
40 Grinder
50 Dehydrator
60 Stirrer
70 Membrane (filter)
120 Culture and degradation vessel
121 Reaction liquid

## Claims

1. A lignocellulose degradation system for degrading lignocellulosic biomass, the lignocellulose degradation system comprising:
at least one of a selection means for selecting a rumen fluid derived from a ruminant using abundance of a specific bacterium as an indicator, a culture vessel for controlling culture of rumen microorganisms using abundance of a specific bacterium as an indicator, or a degradation vessel for controlling degradation of lignocellulose using abundance of a specific bacterium as an indicator; and
a methane fermenter for performing methane fermentation using as a substrate a lignocellulose degradation product produced by degrading lignocellulosic biomass using at least one of the rumen fluid selected in the selection means, a culture fluid in the culture vessel, or a reaction liquid in the degradation vessel.

2. The lignocellulose degradation system according to claim 1, wherein the specific bacterium is at least one or more types of three types of bacteria, which are *Fibrobacter succinogenes, Ruminococcus albus,* and *Prevotella ruminicola.*

3. The lignocellulose degradation system according to claim 1 or 2, wherein abundance of the specific bacterium as measured by a quantitative PCR method is 1.0 × 10⁹ copies/mL or more for *Fibrobacter succinogenes,* 1.0 × 10⁷ copies/mL or more for *Ruminococcus albus,* and 1.0 × 10¹⁰ copies/mL or more for *Prevotella ruminicola.*

4. The lignocellulose degradation system according to any one of claims 1 to 3, wherein at least one of the culture of rumen microorganisms in the culture vessel or the degradation of lignocellulosic biomass in the degradation vessel is controlled based on a parameter including temperature, pH, oxidation-reduction potential (ORP), hydraulic retention time (HRT) or sludge retention time (SRT), and ammonium nitrogen concentration in a culture medium.

5. The lignocellulose degradation system according to any one of claims 1 to 4, wherein the culture vessel or the degradation vessel is a culture and degradation vessel for performing the culture of rumen microorganisms and the degradation of lignocellulose in one vessel.

6. A method for degrading lignocellulose for degrading lignocellulosic biomass, the method comprising:
at least one of (a) selecting a rumen fluid derived from a ruminant using abundance of a specific bacterium as an indicator, (b) controlling culture of rumen microorganisms using abundance of a specific bacterium as an indicator, or (c) controlling degradation of lignocellulose using abundance of a specific bacterium as an indicator; and
(d) performing methane fermentation using as a substrate a lignocellulose degradation product produced by degrading lignocellulosic biomass using at least one of the rumen fluid selected in step (a), a culture fluid obtained in step (b), or a reaction liquid obtained in step (c).
